**Europäisches Patentamt**

**European Patent Office**

**Office européen des brevets**

(11) Veröffentlichungsnummer: **0 410 318 A2**

# (12) EUROPÄISCHE PATENTANMELDUNG

(21) Anmeldenummer: 90113927.9

(22) Anmeldetag: **20.07.90**

(51) Int. Cl.⁵: **A61H 33/04**, A61M 3/02, A61F 11/00

(30) Priorität: 26.07.89 AT 1797/89
08.09.89 DE 3929964

(43) Veröffentlichungstag der Anmeldung:
**30.01.91 Patentblatt 91/05**

(84) Benannte Vertragsstaaten:
**AT BE CH DE DK ES FR GB IT LI LU NL SE**

(71) Anmelder: **SIEMENS & CO.CO.**
**Kaiserstrasse 62**
**D-6500 Mainz(DE)**

(72) Erfinder: **Pechatschek, Hans**
**Agergasse 12**
**A-4880 St. Georgen i. Attg.(AT)**

(74) Vertreter: **Weber, Dieter, Dr. et al**
**Dr. Dieter Weber und Dipl.-Phys. Klaus**
**Seiffert Patentanwälte**
**Gustav-Freytag-Strasse 25 Postfach 6145**
**D-6200 Wiesbaden 1(DE)**

(54) **Nasen- und Ohrendusche.**

(57) Die vorliegende Erfindung betrifft eine Nasen-
oder Ohrendusche, bestehend aus einem Behälter
(1) für Spülflüssigkeit, der als Austrittsöffnung ein
vom Behälter (1) ausgehendes Rohr (5) aufweist.
Um eine Nasen- bzw. Ohrendusche zu schaffen,
welche ohne Risiko und funktionssicher auch von
Laien angewendet werden kann und dabei in ihrem
Aufbau möglichst einfach sowie preiswert herzustellen ist, wird erfindungsgemäß vorgeschlagen, daß
das Rohr (5) an einem beweglichen Ventilkörper
ansetzt und mit diesem verschwenkbar ist, wobei
unterschiedliche Schwenkpositionen dia Offen- und
die Schließstellung des Ventils (3) definieren.

Fig. 1

EP 0 410 318 A2

## NASEN- UND OHRENDUSCHE

Die vorliegende Erfindung betrifft eine Nasen- bzw. Ohrendusche, bestehend aus einem Behälter für Spülflüssigkeit, der als Austrittsöffnung ein vom Behälter ausgehendes Rohr hat. Derartige Behälter bestehen im allgemeinen aus Kunststoff, wobei das Rohr durch eine Öffnung in dem Behälter bzw. in einem Schraubdeckel des Behälters hindurch und in die Flüssigkeit hineinragt, und die Flüssigkeit durch Druck auf die nachgebenden Behälterwände aus dem Rohr herausgepreßt wird. Solche Behälter werden auch als "Spritzflasche" bezeichnet. Speziell für die Spülung von Nase und Ohren gibt es daneben noch aufwendigere Geräte, wie sie in der medizinischen Praxis verwendet werden, die jedoch einen vergleichsweise komplizierten technischen Aufbau aufweisen und weder ohne weiteres in einer Bereitschaftstasche mitführbar sind noch von Laien in Eigenbehandlung angewendet werden können.

Die bekannten Spritzflaschen haben jedoch den Nachteil, daß sich der Druck, mit welchem die Flüssigkeit aus dem Behälter austritt, ebenso wie die austretende Flüssigkeitsmenge nur sehr schlecht kontrollieren und dosieren lassen. Außerdem ist die im Behältar befindliche Flüssigkeit über das offene Rohr immer mit der Umgebungsluft verbunden und so unter Umständen Kontaminationen ausgesetzt.

Schließlich ist das vom Behälter ausgehende Spritzrohr im allgemeinen nur begrenzt beweglich und läßt sich deshalb schlecht in die Nasenöffnungen bzw. den Gehörgang einführen, so daß hierbei der Behälter verdreht werden muß, so daß das innere Rohrende unter Umständen nicht mehr in die Flüssigkeit ragt, was die Anwendung derartiger Flaschen für einen solchen Verwendungszweck erschwert.

Demgegenüber liegt der vorliegenden Erfindung die Aufgabe zugrunde, eine Nasen- bzw. Ohrendusche zu schaffen, welche ohne Risiko und funktonssicher auch von Laien angewendet werden kann und dabei in ihrem Aufbau möglichst einfach sowie preiswert herzustellen ist.

Diese Aufgabe wird dadurch gelöst, daß das Rohr an einem beweglichen Ventilkörper ansetzt und mit diesem verschwenkbar ist, wobei unterschiedliche Schwenkpositionen die Offen- und die Schließstellung des Ventils definieren.

Der Behälter weist also ein Ventil auf, wobei der bewegliche Ventilkörper dieses Ventils unmittelbar mit dem vom Behälter ausgehenden Rohr verbunden und mit diesem verschwenkbar ist. Durch gezieltes Festlegen der Schwenkpositionen für die Offen- bzw. Schließstellung des Ventils läßt sich somit erreichen, daß in einer vorgegebenen Stellung des Rohres, wie sie für den vorgesehenen Spülzweck optimal ist, das Ventil geöffnet ist.

Dabei ist in der bevorzugten Ausführungsform der Erfindung vorgesehen, daß der Behälter einen um ca. 90° abgewinkelten Hals aufweist, an dessen freiem Ende das Ventil angebracht ist. Durch die Abwinkelung des Behälterhalses, an welchem das Ventil ansetzt, kann man in einfacher Weise erreichen, daß das Rohr in einem Bereich um eine Mittenposition herum verschwenkbar ist, welche in etwa der Achse des abgewinkelten Behälterhalses entspricht. Hierdurch ist es möglich, den Behälter so zu drehen, daß der Behälterhals nach unten weist, so daß das am Behälterhals angebrachte Ventil von innen her mit Flüssigkeit beaufschlagt ist und dennoch über das Verschwenken des Rohres die Flüssigkeit in der gewünschten Richtung gelenkt werden kann.

Zweckmäßigerweise ist das Ventil ein Kugelventil und das Rohr verläuft in Fortsetzung einer diametral durch die den beweglichen Ventilkörper bildende Ventilkugel hindurchgehenden Öffnung.

Dabei hat in der bevorzugten Ausführungsform das Kugelventil eine mit dem Behälterhals abgedichtet verbindbare Kappe, welche zur Aufnahme der Ventilkugel eine sphärische Innenfläche aufweist, wobei die die sphärische Innenfläche bildende Wand eine Öffnung zum Behälterinneren hat sowie eine äußere Öffnung, die kleiner als der Durchmesser der Kugel aber größer als der Außendurchmesser des an der Kugel ansetzenden Rohres ist. Die Ventilkugel ist damit in der sphärischen Innenfläche der Ventilkappe in jede Richtung schwenkbar, wobei jedoch die Schwenkbewegungen begrenzt werden durch das an den Rand der äußeren Öffnung anschlagende und an der Kugel ansetzende Rohr, welches aus diesem Grunde einen kleineren Außendurchmesser hat als die äußere Öffnung der Ventilkappe. Der Durchmesser dieser äußeren Öffnung braucht nur wenig kleiner zu sein als der Durchmesser der Kugel, um dieser einen festen Sitz zu geben und um andererseits jedoch auch das Eindrücken der Ventilkugel in die Kappe bzw. die sphärische Innenfläche zu ermöglichen, wobei die Ränder der Öffnung elastisch weggedrückt werden. Außerdem braucht diese Öffnung auch nicht kreisrund zu sein, sondern kann gegebenenfalls Ausbuchtungen oder Fortsätze aufweisen, die den Durchmesser der Öffnung nur stellenweise soweit reduzieren, daß die Ventilkugel sicher in der sphärischen Innenfläche gehalten wird.

Dabei weist der Rand dieser äußeren Öffnung vorzugsweise Aussparungen für die Aufnahme des Rohres zum Festlegen einer definierten Schwenkposition auf. Wenn also das Rohr mit der Ventilkugel so verschwenkt wird, daß es in einer der Aussparungen an Rand der äußeren Öffnung anliegt, so ist durch diese Schwenkposition beispielsweise eine vollständige Offenstellung des Ventils definiert, während die Position des Rohres in einer anderen, ähnlichen Aussparung beispielsweise eine vollständige Schließstellung definieren kann. Dabei können auch mehrere, unterschiedliche Schwenkpositionen jeweils eine Offen- bzw. eine Schließstellung definieren.

Dabei ist erfindungsgemäß vorgesehen, daß die definierten Schwenkpositionen jeweils maximalen Auslenkungen parallel bzw. senkrecht zur Längserstreckung des Behälters entsprechen.

Beispielsweise hat also der Rand der äußeren Öffnung vier paarweise einander gegenüberliegende Aussparungen zur Aufnahme des Rohres, wobei eines dieser Paare im wesentlichen senkrecht und das andere im wesentlichen parallel zur Achse bzw. zur Längserstreckung des Behälters ausgerichtet sind.

Die innere Öffnung des Ventils bzw. der die sphärische Innenfläche bildenden Wand ist vorzugsweise ein länglicher Schlitz. Dieser Schlitz kann im wesentlichen parallel oder senkrecht zur größten Längserstreckung des Behälters verlaufen. Verläuft der Schlitz, wie in der bevorzugten Ausführungsform, parallel zur Längserstreckung des Behälters und ist auch ein Paar von Aussparungen vorgesehen, welches in etwa in dieser Richtung angeordnet ist, so liegt die nach innen gerichtete Öffnung der Bohrung der Ventilkugel im Bereich dieses Schlitzes, wenn sich das Rohr in den Aussparungen befindet, die ebenfalls in Längsrichtung des Behälters, also parallel zum Schlitz der inneren Öffnung ausgerichtet sind. Wird dagegen das Rohr in quer dazu angeordnete Aussparungen verschwenkt, so liegt die nach innen gerichtete Öffnung der Bohrung der Ventilkugel im Bereich der sphärischen Innenfläche, ohne mit dem Schlitz zu überlappen. Hierdurch ist also die Schließstellung des Ventils definiert, während die zuvor beschriebenen Positionen eine Offenstellung definieren.

Weiterhin ist in der bevorzugten Ausführungsform der Erfindung vorgesehen, daß der Behälter auf seiner dem Ventil abgewandten Seite einen Schraubverschluß aufweist. Der Behälter kann damit ohne Lösen oder Öffnen des Ventils bzw. der Ventilkappe gefüllt und gegebenenfalls auch entleert werden.

Bevorzugt ist weiterhin eine Ausführungsform der Erfindung, bei welcher der Deckel des Schraubverschlusses eine mit dem Finger verschließbare Öffnung hat. Ein solcher Behälter wird mit geschlossenem Ventil und abgeschraubtem Deckel des Schraubver schlusses gefüllt. Anschließend wird der Deckel des Schraubverschlusses aufgeschraubt. Der Schraubverschluß befindet sich dabei oben, während der gegebenenfalls abgewinkelte Behälterhals mit dem Ventil sich unten befindet, so daß das Ventil mit dem hydrostatischen Druck der Flüssigkeit in dem Behälter beaufschlagt ist. Wird nun die Öffnung im Schraubdeckel des Behälters mit dem Finger verschlossen, so kann das Ventil in die Offenstellung gebracht werden, ohne daß Flüssigkeit aus dem Ventil austritt, da der äußere Luftdruck dem hydrostatischen Druck der Flüssigkeit entgegenwirkt und ein Druckausgleich im Inneren des Behälters nicht stattfinden kann, solange die Deckelöffnung mit dem Finger verschlossen wird. Nun kann das Rohr oder gegebenenfalls eine auf das Rohr aufgesetzte Düse an einem Nasenloch oder am Gehörgang angesetzt werden. Dann wird die Deckelöffnung freigegeben, so daß im Inneren des Behalters ein Druckausgleich stattfinden kann, und die Flüssigkeit tritt aus dem Ventil aus und in die Nase bzw. den Gehörgang ein. In vorteilhafter Weise hat man hierbei einen durch die Höhe des Flüssigkeitsspiegels bezüglich der Rohröffnung eindeutig definierten Druck, so daß insbesondere nicht die Gefahr besteht, daß Flüssigkeit mit zu hohem Druck aus dem Ventil austritt und so möglicherweise Verletzungen und/oder Schmerzen hervorruft.

Außerdem ist zur weiteren Vereinfachung der Handhabung vorgesehen, daß zwei gegenüberliegende Seitenteile des Behälters in im wesentlichen parallelen Ebenen verlaufen und eine zur Achse des Behälters quer verlaufende Riffelung aufweisen. Ein solcher Behälter kann sehr einfach in eine Halterung eingeschoben werden, die im wesentlichen aus zwei im Abstand der Seitenwände voneinander angeordneten, vorstehenden Fingern besteht. Der Behälter wird dann zwischen diese Finger eingeschoben, wobei die Riffelungen ein Verrutschen des Behälters verhindern.

Das von dem Ventil ausgehende Rohr kann entweder selbst als Düse ausgebildet sein oder es können auf das Rohr aufsteckbare Düsen vorgesehen sein. Letzteres ist insbesondere dann zu bevorzugen, wenn entweder verschiedene Personen dieselbe Nasen- bzw. Ohrendusche benutzen sollen und/oder wenn diese sowohl als Nasendusche, als auch als Ohrendusche verwendet werden soll, wobei aus anatomischen Gründen selbstverständlich verschiedene Düsenformen verwendet werden sollten.

Eine bevorzugten Ausführungsform der Erfindung wird nun mit ihren Vorteilen, Merkmalen und Anwensungsmöglichkeiten anhand der dazugehörigen Zeichnungen beschrieben. Es zeigen:

Figur 1 die Seitenansicht einer Nasendusche, bestehend aus Behälter mit Schraubdeckel und Kugelventil,

Figur 2 eine teilweise Schnittansicht des Behälterhalses mit Kugelventil, und

Figurren 3a-c verschiedene Ansichten, teilweise im Schnitt, der Kappe des Kugelventils.

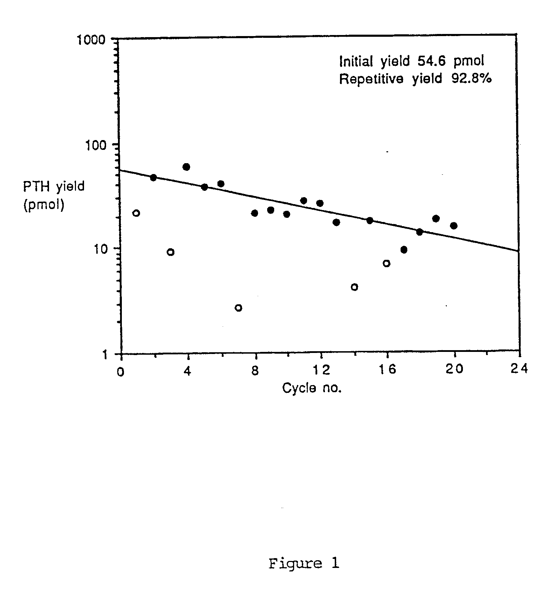

In Figur 1 erkennt man einen im wesentlichen zylindrischen Behälter 1, der oben einen Schraubverschluß 14 mit Schraubdeckel 15 aufweist und an dessen unterem Ende ein Hals 2 angesetzt ist, der um etwa 90° abgewinkelt ist, wobei auf den Rand der so abgewinkelten Halsöffnung die Kappe 4 eines Kugelventils 3 aufgesetzt ist. Abweichend von der zylindrischen Grundform hat der Behälter 1 zwei gegenüberliegende ebene Seitenteile 16 mit einer Riffelung 17, wobei in Figur 1 nur eines dieser beiden Seitenteile 16 sichtbar ist.

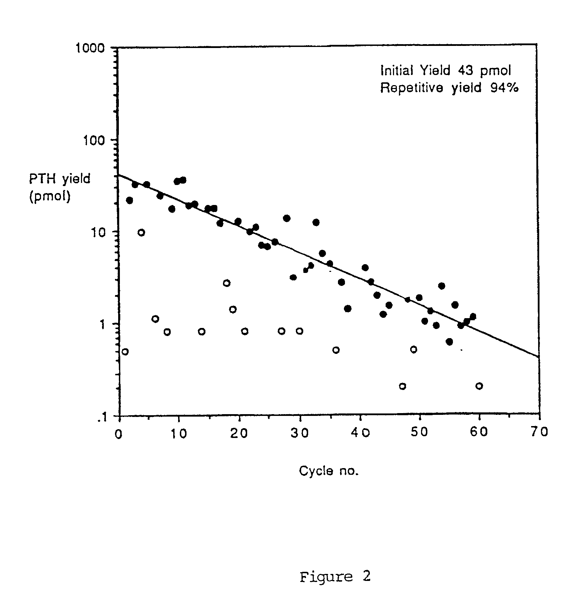

Wie man auch in Figur 2 erkennt, hat die Ventilkappe 4 eine sphärische Innenfläche, in welcher eine durchbohrte Ventilkugel 6 gelagert ist. In Verlängerung der Bohrung 7 der Ventilkugel 6 ist eir Rohr 5 angesetzt, welches zumindest an seiner Außenseite leicht konisch ist. Auf das konische Rohr 5 können verschiedene Düsen 18, 18′ aufgesetzt werden, wobei die Düse 18 für eine Nasenspülung vorgesehen ist, während die nur gestrichelt und in einer anderen Schwenkposition der Kugel 6 dargestellte Düse 18′ für die Spülung eines Gehörganges vorgesehen ist.

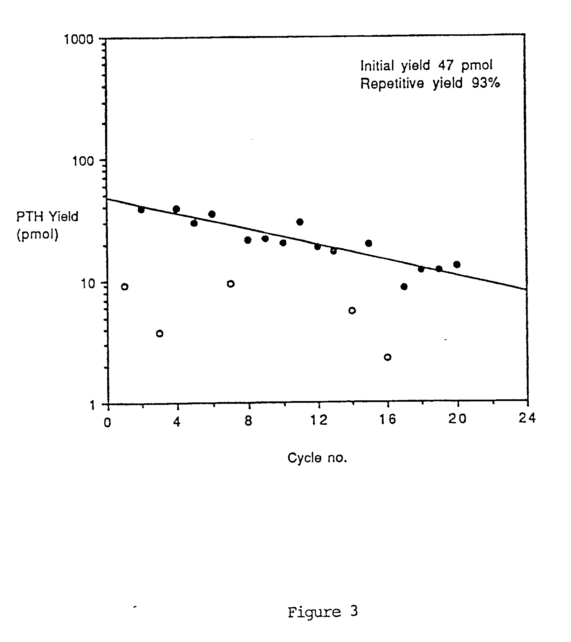

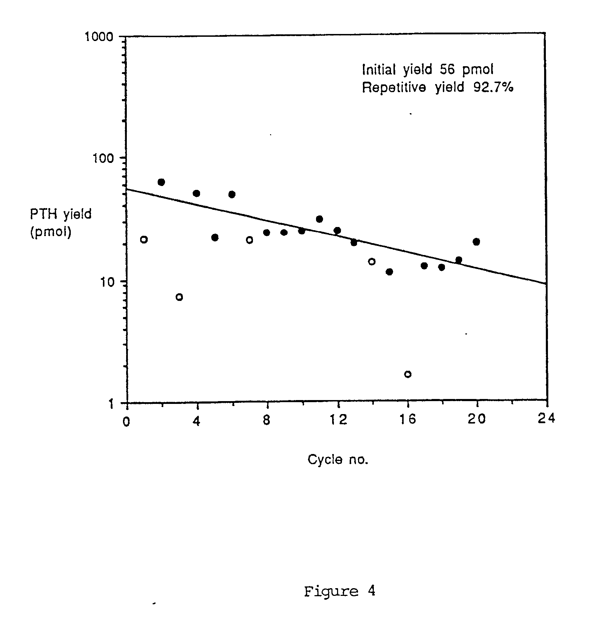

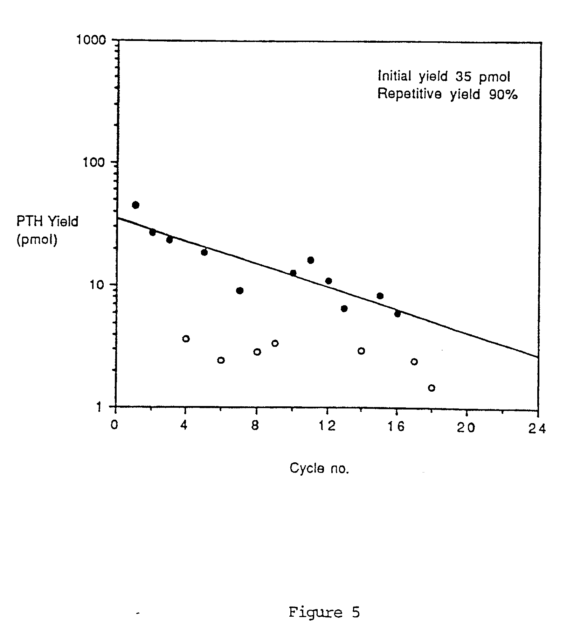

Das Zusammenwirken der Ventilkugel 6 mit der sphärischen Innenfläche 8 der Ventilkappe 4 erkennt man besser aus den prinzipiellen Darstellungen der Kappe in den Figuren 3a bis c. Dabei sind die Figuren 3a und b aufeinander senkrecht stehende Schnitte, welche die Achse der Kappe enthalten und parallel bzw. senkrecht zur Erstreckung des Schlitzes 10 verlaufen, welcher die innere Öffnung der Wand 9 der Kappe 4 bildet. Figur 3a entspricht dabei der Lage der Kappe, wie sie auch in Figur 2 dargestellt ist. Die Kappe 4 hat einen ringförmigen Wulstrand 19, welcher einen entsprechend gestalteten Rand der Öffnung des Behälterhalses dichtend hintergreift. Der Ventilteil dieser Kappe besteht im wesentlichen aus einer Wand 9 mit einer sphärischen Innenfläche 8, wobei diese Wand 9 bzw. die sphärische Innenfläche 8 eine äußere Öffnung 11 und eine als Schlitz 10 gestaltete innere Öffnung aufweisen. Die Ventilkugel 6 liegt dichtend an der sphärischen Innenfläche an, wobei bei entsprechender Lage der Ventilkugel über eine diametral durch die Kugel hindurchgehende Bohrung 7 der Schlitz 10 mit der äußeren Öffnung 11 verbunden wird. Als Fortsetzung der diametralen Bohrung 7 der Ventilkugel 6 ist ein Rohr 5 vorgesehen, welches innerhalb der äußeren Öffnung 11 durch Verschwenken der Ventilkugel 6 begrenzt bewegbar ist.

Figur 3c ist eine schematische Ansicht der Ventilkappe 4 vom Behälterhals her gesehen. Man erkennt außen den Wulstrand 19 und strichpunktiert angedeutet den Durchmesser der Ventilkugel 6. In der Wand 9 befindet sich ein Schlitz 10, dessen Breite in etwa dem Durchmesser der diametralen Bohrung 7 der Ventilkugel entspricht. Außerdem sind in Figur 3c gestrichelt zwei verschiedene Positionen der Bohrung 7 angedeutet, die man durch entsprechendes Verschwenken der Ventilkugel 6 mit dem Rohr 5 erreicht. In der Position 20, die auch der in Figur 2 dargestellten Position entspricht, fällt die innere Öffnung der Bohrung 7 mit dem unteren Ende des Schlitzes 10 zusammen, so daß Flüssigkeit in die Bohrung 7 ein- und aus dem Rohr 5 austreten kann. In der Position 21 ist das Rohr 5 in horizontaler Richtung verschwenkt, so daß die innere Öffnung der Bohrung 7 an der Wand 9 der sphärischen Innenfläche 8 anliegt, ohne mit dem Schlitz 10 zu überlappen. In dieser Stellung ist das Ventil geschlossen.

Wie man in den Figuren 1, 3a und 3b erkennt, weist der Rand 12 der äußeren Öffnung 11 Aussparungen auf, die eine maximale Auslenkung des Rohres 5 in Richtung dieser Aussparungen erlauben. Es sind jeweils gegenüberliegende Paare von Aussparungen 13 vorgesehen, wobei eines dieser Paare im wesentlichen vertikal und das andere horizontal ausgerichtet ist. Der Rand 12 der Öffnung 11 erscheint dadurch gewellt. Die Schließstellungen des Ventils sind dabei im wesentlichen dadurch definiert, daß sich das Rohr in einer der horizontal ausgerichteten Aussparungen befindet, während in den übrigen Rohrstellungen, insbesondere auch in der Mittenstellung, d.h. der Ausrichtung des Rohres entlang der Ventilkappenachse, das Ventil 3 geöffnet ist.

Es versteht sich, daß durch entsprechende Ausrichtung des Schlitzes oder durch eine andersartige Gestaltung der inneren Öffnung der Wand der Ventilkappe beliebige Stellungen des Rohres in dem durch die äußere Öffnung vorgegebenen Schwenkbereich als Schließ- bzw. Offenstellung festgelegt werden können.

Der Deckel 15 des Schraubverschlusses 14 weist an seiner Oberseite eine in den Zeichnungen nicht sichtbare Öffnung mit einem Durchmesser von etwa 2 bis 8 mm auf, welche leicht mit einem Finger verschlossen werden kann.

Die Düsen 18, 18′ haben einen Innenkonus, der auf das Rohr 5 paßt, wobei die Düse 18 im Bereich ihrer Öffnung eine kugelartige Verdickung aufweist, die es ermöglicht, die Düse im wesentlichen rundum dichtend an ein Nasenloch anzusetzen, so daß bei einer Nasenspülung die Flüssigkeit nicht vorbeifließt.

Die Düse 18′ für eine Ohrenspülung ist dagegen spitz zulaufend gestaltet, so daß sie leicht in den Gehörgang eingeführt werden kann.

Die Flasche kann mit ihren gegenüberliegenden Seitenteilen 16 leicht in eine Wandhalterung eingeschoben werden, die im wesentlichen aus zwei im Abstand der Seitenteile 16 angeordneten hervorstehenden Fingern besteht, welche die Seitenteile zwischen den Riffelungen 17 ergreifen, wobei die Riffelungen 17 ein Abrutschen des Behälters 1 verhindern.

Eine solche Nasen- bzw. Ohrendusche läßt sich problemlos mit Flüssigkeit befüllen und gefahrlos und sicher auch von Laien anwenden.

**Ansprüche**

1. Nasen- oder Ohrendusche, bestehend aus einem Behälter (1) für Spülflüssigkeit, der als Austrittsöffnung ein vom Behälter (1) ausgehendes Rohr (5) aufweist, dadurch gekennzeichnet, daß das Rohr (5) an einem beweglichen Ventilkörper ansetzt und mit diesem verschwenkbar ist, wobei unterschiedliche Schwenkpositionen die Offen- und die Schließstellung des Ventils (3) definieren.

2. Dusche nach Anspruch 1, dadurch gekennzeichnet, daß der Behälter (1) einen um ca. 90° abgewinkelten Hals (2) aufweist, an dessen freiem Ende das Ventil (3) angebracht ist.

3. Dusche nach Anspruch 1 oder 2, dadurch gekennzeichnet, daß das Ventil (3) ein Kugelventil ist und das Rohr (5) in Fortsetzung einer diametral durch die den beweglichen Ventilkörper bildende Ventilkugel (6) hindurchgehenden Bohrung (7) verläuft.

4. Dusche nach Anspruch 3, dadurch gekennzeichnet, daß das Kugelventil eine mit dem Hals (2) abgedichtet verbindbare Kappe (4) aufweist, welche zur Aufnahme der Ventilkugel (6) eine sphärische Innenfläche (8) aufweist, wobei die die sphärische Innenfläche (8) bildende Wand (9) eine Öffnung zum Behälterinneren hat, sowie eine äußere Öffnung (11), die kleiner als der Durchmesser der Ventilkugel und größer als der Außendurchmesser des an der Ventilkugel (6) ansetzenden Rohres (5) ist.

5. Dusche nach Anspruch 4, dadurch gekennzeichnet, daß der Rand (12) der äußeren Öffnung (11) Aussparungen (13) für die Aufnahme des Rohres (5) zum Festlegen einer definierten Schwenkposition aufweist.

6. Dusche nach Anspruch 5, dadurch gekennzeichnet, daß die definierten Schwenkpositionen jeweils maximalen Auslenkungen parallel bzw. senkrecht zur größten Längserstreckung des Behälters (1) entsprechen.

7. Dusche nach einem der Ansprüche 4 bis 6, dadurch gekennzeichnet, daß die innere Öffnung ein länglicher Schlitz (10) ist.

8. Dusche nach Anspruch 7, dadurch gekennzeichnet, daß der Schlitz (10) parallel zur größten Längserstreckung des Behälters (1) verläuft.

9. Dusche nach einem der Ansprüche 1 bis 8, dadurch gekennzeichnet, daß der Behälter (1) auf der dem Ventil (3) abgewandten Seite einen Schraubverschluß (14) aufweist.

10. Dusche nach Anspruch 9, dadurch gekennzeichnet, daß der Deckel (15) des Schraubverschlusses (14) eine mit dem Finger verschließbare Öffnung hat.

11. Dusche nach einem der Ansprüche 1 bis 10, dadurch gekennzeichnet, daß zwei gegenüberliegende Seitenteile (16) des Behälters (1) in im wesentlichen parallelen Ebenen verlaufen und eine zur Längserstreckung des Behälters quer verlaufende Riffelung (17) aufweisen.

12. Dusche nach einem der Ansprüche 1 bis 11, dadurch gekennzeichnet, daß das Rohr (3) als Düse ausgebildet oder eine auf das Rohr (3) aufsteckbare Düse (18, 18') vorgesehen ist.

Figure 1

Figure 2

Figure 3

Figure 4

9

Figure 5